# FASCICULE DE BREVET EUROPEEN

(11) **EP 0 783 483 B1**
(45) Date de publication et mention de la délivrance du brevet: **09.06.1999**
(21) Numéro de dépôt: 94922292.1
(22) Date de dépôt: 22.07.1994
(51) Int. Cl.: C07C 215/76, C07C 215/86, C07D 498/10, C07D 209/08, G03C 1/685

(54) **PROCEDE DE PREPARATION DE COMPOSES PHOTOCHROMIQUES DU TYPE SPIRO[INDOLINE-[2,3']-BENZOXAZINE] ANNELE**
VERFAHREN ZUR HERSTELLUNG VON PHOTOCHROMEN ANNELIERTEN SPIRO[INDOLIN-[2,3']-BENZOXAZIN]VERBINDUNGEN
METHOD FOR PREPARING PHOTOCHROMIC COMPOUNDS OF THE SPIRO[INDOLIN-[2,3']-BENZOXAZINE] RINGED TYPE

(43) Date de publication de la demande: 16.07.1997
(73) Titulaire: ESSILOR INTERNATIONAL (COMPAGNIE GENERALE D'OPTIQUE), F-94227 Charenton cédex (FR)
(72) Inventeur: PALTCHKOV, Victor A., Rostov on Don, 344044 (RU); CHELEPIN, Nikolai E., Rostov on Don, 344007 (RU); MINKIN, Vladimir I., Rostov on Don, 344006 (RU); TROFIMOVA, Nadezhda S., Rostov on Don, 344092 (RU); ZOUBKOV, Oleg A., Rostov on Don, 344090 (RU)
(74) Mandataire: Casalonga, Axel
(86) Numéro de dépôt international: FR9400918
(87) Numéro de publication internationale: WO9603368

(56) Documents cités:
- CHEMICAL ABSTRACTS, vol. 111, no. 5, 31 Juillet 1989, Columbus, Ohio, US; abstract no. 39378q, SHINICHI YAMAMOTO ET AL 'Preparation of spirooxazine compounds from indolenium salt nitrous acid and aminohydroxyarenes' page 604 ; & JP,A,63 301 885 (TORAY INDUSTRIES) 8 Décembre 1988

## Description

La présente invention concerne un nouveau procédé de préparation de composés photochromiques du type spiro[indoline-[2,3']-benzoxazine annelé.

Le photochromisme est un phénomène réversible bien connu, illustré par exemple par un composé qui change de couleur quand il est exposé à des radiations lumineuses dont certaines appartiennent au domaine de l'U.V., notamment les radiations solaires, et qui reprend sa couleur initiale lorsqu'on interrompt l'exposition lumineuse.

De tels composés sont utilisés par exemple dans la fabrication de lentilles pour lunettes de protection solaire ou dans d'autres applications où intervient le besoin de faire varier la transparence d'un article en fonction de l'intensité lumineuse environnante. ns peuvent être appliqués sur un support transparent ou incorporés dans un matériau organique polymérisé transparent en combinaison avec une très grande variété de compositions polymériques.

Un certain nombre de composés photochromiques organiques comportant dans leur formule un groupe indolino-spiro-oxazine ont déjà été proposés pour de telles applications, notamment dans le domaine des lentilles ophtalmiques.

Ainsi, les brevets américains US-3.562.172 et 3.578.602 ont divulgué l'effet photochromique de certains composés appartenant à la famille des indolino-spironaphtoxazines, tandis que le brevet américain US-4.215.010 décrit des indolino-spironaphtoxazines dont le cycle naphtalène est substitué par des groupements méthoxy, éthoxy ou un atome d'halogène. Des composés photochromiques analogues comportant un cycle pyridobenzène au lieu du cycle naphtalène des composés ci-dessus, sont décrits dans le brevet américain US-4.720.547, ainsi que dans la demande internationale WO 87/00524.

Cependant, les composés décrits dans cet art antérieur ne satisfont pas pleinement à l'ensemble des qualités que l'on en attend. Si l'on se place plus particulièrement dans le cadre de l'application aux lentilles de protection solaire, le matériau photochromique obtenu, que ce soit à partir d'une solution du composé photochromique ou par incorporation de celui-ci dans un polymère organique, doit présenter un certain nombre de propriétés concernant l'effet photochromique, en plus de sa transparence naturelle et de sa compatibilité avec les matériaux couramment utilisés pour de telles lentilles.

En particulier :
- Quand il est irradié dans son domaine de photosensibilité, l'apparition de la coloration doit apparaître rapidement, en un temps de préférence de l'ordre de la seconde, et sa disparition quand l'irradiation cesse doit être tout aussi rapide.
- Le matériau doit être stable dans le temps, aussi bien par lui-même que dans son photochromisme. Ainsi, le composé doit pouvoir supporter au sein du matériau, un nombre élevé de cycles coloration-décoloration, tout au long d'une durée d'utilisation qui peut être de plusieurs années.
- La colorabilité doit être bonne pour des teneurs en composé photochromique raisonnables, et le spectre d'absorption du matériau irradié doit couvrir autant que possible l'ensemble du spectre visible.
- L'effet photochromique doit être, de préférence, indépendant du substrat renfermant le composé, et il doit se manifester dans une plage de températures étendue, tant à des températures ambiantes variables que quand il est échauffé sous irradiation.
- La coloration prise par le matériau sous irradiation doit aussi répondre à des soucis d'ordre esthétique, en préservant une vision de l'environnement agréable pour le porteur de lunettes ou lentilles ophtalmiques en de tels matériaux. De ce point de vue, il convient d'éviter les couleurs bleues auxquelles conduisent généralement les composés de l'art antérieur précité, et de privilégier le vert.

Dans le but de satisfaire à ces conditions, on a déjà proposé dans les demandes de brevet français 2.647.789 et 2.647.790 et la demande de brevet européen EP-0245020, des composés photochromiques répondant à la structure spiro[indoline-[2,3']-benzoxazine] annelé. Il s'agit de composés spiro[indoline-[2,3']-benzoxazine] dont le noyau benzénique de la partie benzoxazine est orthocondensé par un noyau aromatique hétérocycle ou non.

Ces composés sont obtenus par un procédé comportant une condensation d'une base de Fischer libre du type 2-alkylidène indoline avec un dérivé 1-nitroso 2-phénol annélé.

Selon une variante de ce procédé, on peut également préparer la base de Fischer in situ à partir d'un sel d'indoléninium correspondant, en présence d'une base telle que pipéridine ou triéthylamine.

Le document Chem. Abs. Vol 111, No. 5, 1989 Abstract No. 393 789 décrit un procédé de préparation d'un composé spirooxazine dans lequel on fait réagir le produit de réaction d'un sel d'indolènium et d'acide nitreux avec un aminohydroxyarène.

La demanderesse a découvert de façon inattendue un nouveau procédé de préparation de composés spiro[indoline-[2,3']-benzoxazine] annelé, permettant d'obtenir des rendements en produit final plus élevés que les procédés antérieurs définis ci-dessus pour le même produit final.

Le procédé de l'invention permet également de travailler dans des conditions plus douces que les procédés de l'art antérieur.

Le procédé de l'invention est caractérisé essentiellement par le fait qu'il comporte la réaction d'une base Fischer (I) du type 2-alkylidène indoline sur un composé orthoaminophénol annélé (II) en présence d'un agent oxydant.

Selon une première variante, la base de Fischer, l'orthoaminophénol annélé et l'agent oxydant sont mélangés simultanément pour effectuer la réaction.

Selon une deuxième variante du procédé de l'invention, on fait réagir dans une première étape l'agent oxydant sur la base de Fischer, puis on ajoute au mélange réactionnel ainsi obtenu l'orthoaminophénol annélé.

Selon les différentes formes de procédé de l'invention, la base de Fischer du type 2-alkylidène indoline peut également être formée in situ dans le milieu réactionnel à partir d'un précurseur ayant pour structure un halogénure d'indoléninium correspondant à ladite base de Fischer, en présence d'une base telle que pipéridine ou triéthylamine.

Le composé orthoaminophénol annélé peut également être formé in situ à partir d'un précurseur ayant la structure d'un sel d'addition acide dudit orthoaminophénol annélé, en présence d'une base telle que pipéridine ou triéthylamine.

De manière préférentielle, la réaction de condensation de la base de Fischer avec l'orthoaminophénol annélé est mise en oeuvre dans un milieu solvant. Le solvant utilisé est de préférence le toluène ou le chloroforme.

Les agents oxydants utilisés selon le procédé de l'invention, sont choisis de préférence parmi les oxydes de métalloïde tels que soufre ou selenium et les oxydes de métal de transition tels que le chrome.

On utilise plus préférentiellement le diméthylsulfoxyde, le chlorochromate de pyridinium ou le bioxyde de selenium. Le diméthylsulfoxyde (DMSO) est particulièrement préféré dans la mesure où il permet d'atteindre de meilleurs rendements en produit final.

Les conditions de mise en oeuvre du procédé de l'invention, notamment le choix du solvant, les conditions de température et l'utilisation de certains additifs pour améliorer le rendement en produit final, varient en fonction de la nature de l'agent oxydant.

Lorsqu'on utilise le diméthylsulfoxyde (DMSO), on applique de préférence le procédé de l'invention comprenant le mélange simultané des trois réactifs. On utilise préférentiellement le toluène comme solvant. On utilise plus particulièrement un agent déshydratant tel que le sulfate de magnésium ou le chlorure de calcium et/ou un agent activateur du DMSO comme l'hydrogénocarbonate de sodium.

La température de réaction de condensation varie de préférence entre 50 et 80°C.

Lorsqu'on utilise le bioxyde de selenium, on applique plus particulièrement le procédé comprenant deux étapes, à savoir l'oxydation de la base de Fischer suivie de l'addition de l'orthoaminophénol annélé au milieu réactionnel obtenu. On utilise de préférence le toluène comme solvant. La première étape d'oxydation est réalisée de préférence à température ambiante en milieu solvant. La température de réaction de condensation varie de préférence entre 50 et 100°C.

Lorsqu'on utilise le chlorochromate de pyridinium de formule Py H⁺ClCrO₃-, on applique de préférence le procédé de l'invention dans lequel les réactifs sont mélangés simultanément. Le solvant utilisé est de préférence le chloroforme. On ajoute préférentiellement un réactif basique tel que pipéridine ou triéthylamine dans le milieu réactionnel après l'étape de condensation de la base de Fischer pour neutraliser l'excès d'oxydant.

La température de réaction de condensation est de préférence de l'ordre de la température ambiante.

Selon la présente invention, on synthétise préférentiellement les composés spiro[indoline-[2,3']-benzoxazine], répondant à la formule suivante : 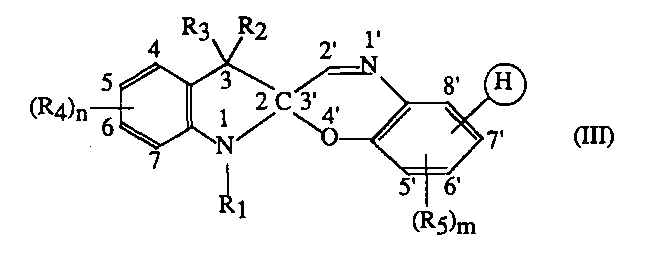 dans laquelle :
m est 1 ou 2;
n varie de 0 à 4;
R₁ représente :
   i) un groupe alkyle de 1 à 16 atomes de carbone, tel que méthyle, éthyle, n-propyle, isopropyle, n-butyle;
   ii) un groupe allyle, phényle, arylalkyle tel qu'un groupe benzyle, phényle mono- ou disubstitué par des substituants du type alkyle ou alcoxy de 1 à 6 atomes de carbone, ou des atomes d'halogène tels que le chlore;
   iii) un groupe alicyclique tel qu'un groupe cyclohexyle éventuellement substitué;
   iv) un groupe hydrocarbure aliphatique comportant dans sa chaîne un ou plusieurs hétéroatomes tels que O, N ou S, notamment une fonction acide, ester ou alcool;

- R₂ et R₃ peuvent, chacun et indépendamment l'un de l'autre, représenter un groupe C₁-C₈ alkyle, phényle, phényle mono- ou disubstitué par des groupes C₁-C₄ alkyle et/ou C₁-C₅ alcoxy ou peuvent être combinés pour former une chaîne cyclique de 6 à 8 atomes de carbone;
- R₄ et R₅ peuvent, chacun et indépendamment l'un de l'autre, représenter :
   i) un atome d'hydrogène, une fonction amine NR'R", où R' et R" représentent chacun et indépendamment un atome d'hydrogène, un groupe alkyle, cycloalkyle, phényle ou un dérivé substitué de celui-ci; R' et R" peuvent se combiner pour former un cycloalkyle pouvant être substitué et contenir un ou plusieurs hétéroatomes;
   ii) un groupe R, OR, SR, COR ou COOR, dans lequel R représente un atome d'hydrogène, un groupe alkyle de 1 à 6 atomes de carbone ou un groupe aryle ou hétéroaryle;
   iii) un atome d'halogène, un groupe C₁-C₄ monohaloalkyle, l'halogène étant notamment Cl ou Br, ou un groupe C₁-C₄ polyhaloalkyle tel que CF₃;
   iv) -NO₂, CN, SCN,
chacun des substituants R₄ pouvant être présent sur l'un quelconque des atomes de carbone convenables de la partie indoline du composé photochromique, en positions 4, 5, 6 et 7, quand l'autre est un atome d'hydrogène, alors que quand n=2, il est préférable que les substituants soient présents en positions 4 et 5, 5 et 6, 4 et 6 ou 6 et 7.
Si m=1, R₅ peut désigner également un cycle benzénique condensé en positions 5', 6';
H désigne un cycle benzénique ou naphtalénique condensé en 7', 8' ou un hétérocycle à 5 ou 6 chaînons comportant 1 à 3 hétéroatomes choisis parmi oxygène, soufre ou azote, pouvant être substitué par un ou plusieurs groupements alkyle, alcoxy, amino, aryle, aralkyle ou condensé par un noyau aromatique.

Les noyaux hétérocycles plus particulièrement préférés sont choisis parmi les noyaux pyrimidinique, pyrazinique, furane éventuellement condensé avec un cycle aromatique pour former un cycle benzofurane, thiazole, éventuellement substitués.

Les composés de formule (III) sont obtenus par le procédé de l'invention en faisant réagir, en présence d'un agent oxydant tel que défini ci-dessus, une base de Fischer de formule : 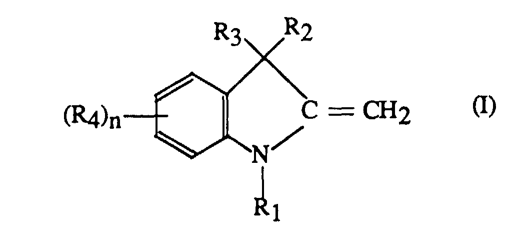 où R₁, R₂, R₃, R₄ et n ont les mêmes significations indiquées ci-dessus, sur un orthoaminophénol annélé de formule suivante : 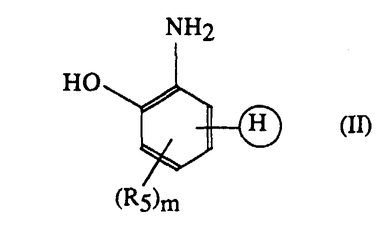 où R₅, m et H ont les mêmes significations indiquées ci-dessus.

Le procédé de préparation permettant d'obtenir les composés de formule (m) peut être représenté par le schéma réactionnel 1 suivant :

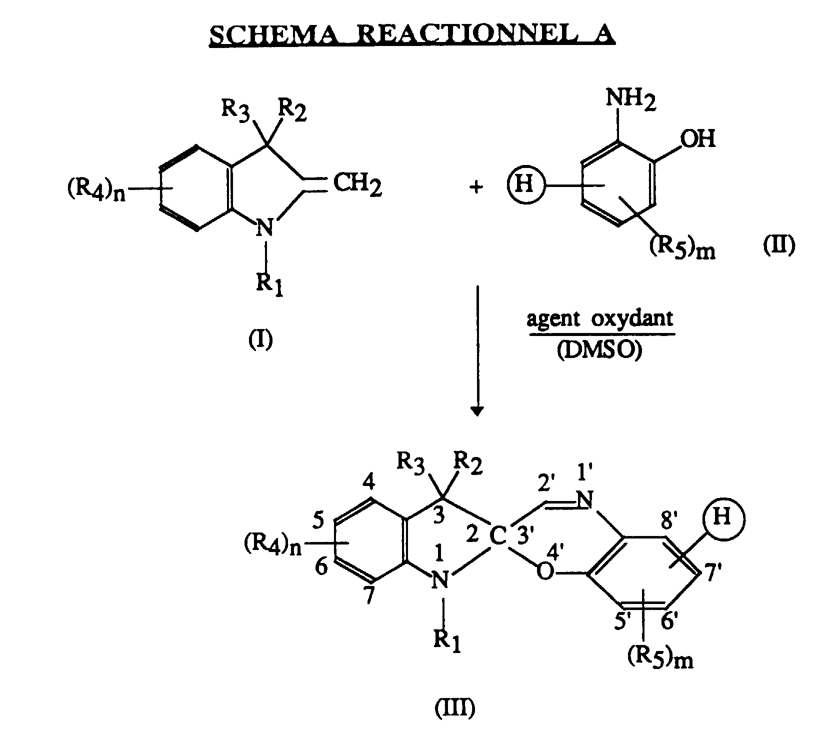

Selon une variante de ce procédé de préparation, on fait réagir dans un premier temps la base de Fischer (I) avec l'agent oxydant et on additionne au mélange réactionnel obtenu l'orthoaminophénol (II) pour obtenir la spirooxazine finale (III).

On peut représenter cette variante par le schéma réactionnel 2 suivant, dans lequel on prévoit la formation d'un produit intermédiaire (IV)

Ce schéma réactionnel ne constitue qu'une explication possible et ne doit pas conduire à une interprétation limitative de l'invention.

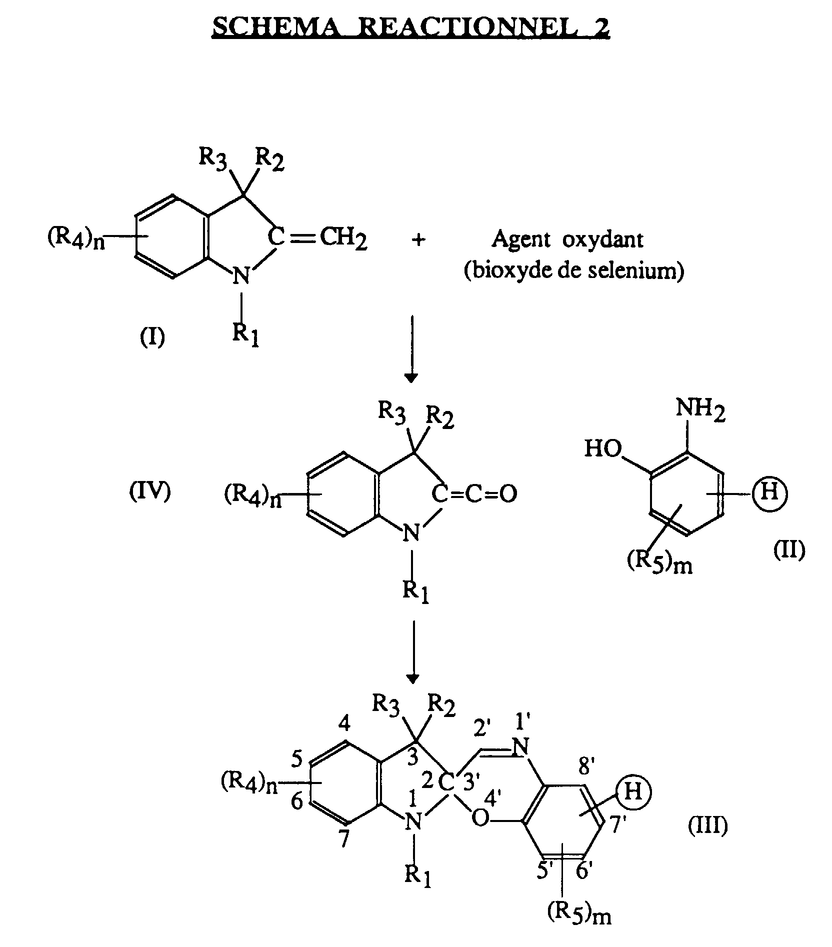

Les composés de formule (I) peuvent être formés in situ dans le mélange réactionnel en présence d'une base telle que triéthylamine ou pipéridine, à partir d'un précurseur ayant pour structure : 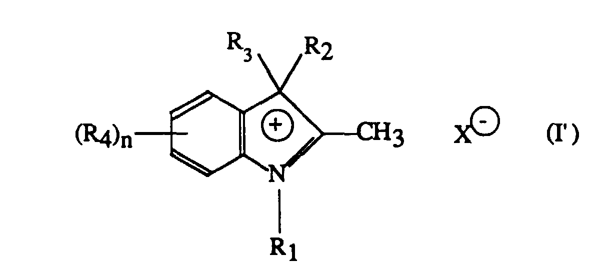 dans laquelle :
R₁, R₂, R₃, R₄ et n ont les mêmes significations que dans la formule (m) indiquée ci-dessus; et
X désigne un atome d'halogène, de préférence l'iode.

Les composés de formule (II) peuvent être formés in situ dans le milieu réactionnel en présence d'une base telle que triéthylamine ou pipéridine, à partir de précurseurs ayant pour structure : 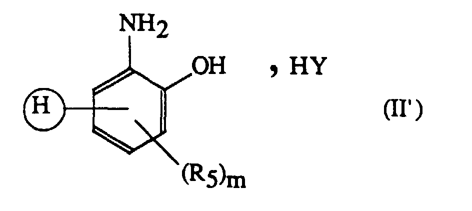 où R₅, m et H ont les mêmes significations indiquées ci-dessus, et Y désigne Cl⁻, Br⁻ ou HSO₄⁻.

Selon la présente invention, on synthétise plus particulièrement avec les procédés tels que définis ci-dessus, les composés de formule : 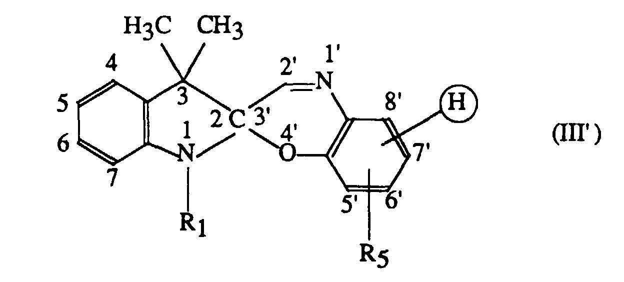 dans laquelle :
R₁ désigne un alkyle en C₁-C₈, benzyle ou 3,4-dichlorobenzyle;
R₅ désigne un atome d'hydrogène, un cycle benzénique condensé en positions 5', 6';
H a la même définition indiquée dans la formule (III) précédente.

Les bases de Fischer utilisées ont pour formule :

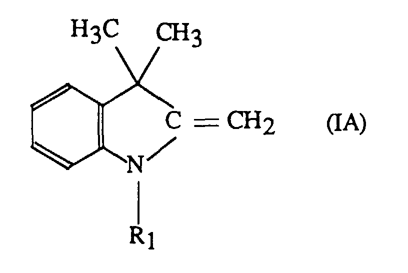

Leurs précurseurs du type halogénure d'indoléninium ont pour formule : 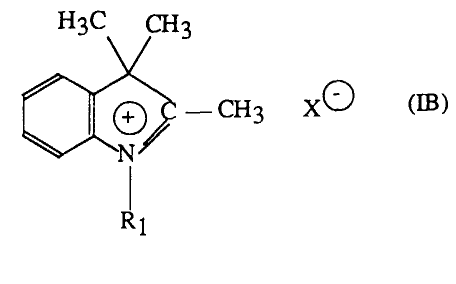 où R₁ a les mêmes significations indiquées ci-dessus, et X désigne un halogène.

Les orthoaminophénols annélés utilisés ont pour formule : 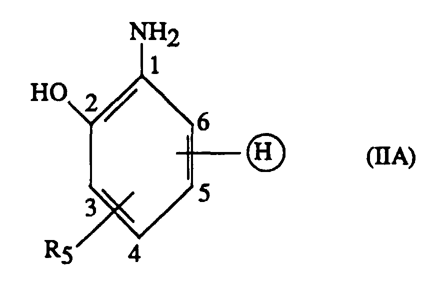 et leurs précurseurs sous forme de sels d'addition d'acides ont pour formule : 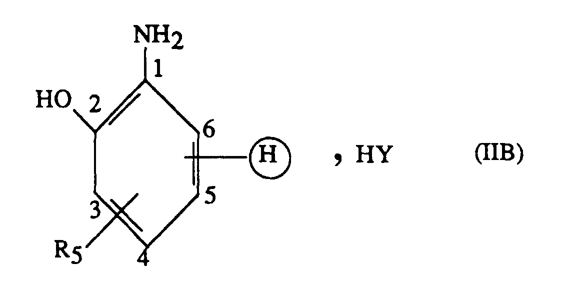 dans lesquelles :
R₅ désigne un atome d'hydrogène ou un noyau benzénique condensé en 3,4;
H désigne un cycle benzénique ou naphtalénique condensé en 5,6 ou un hétérocycle tel que défini dans la formule (III) précédente;
Y désigne Cl⁻, Br⁻ ou HSO₄⁻.

Les exemples qui suivent servent à illustrer l'invention sans toutefois présenter un caractère limitatif.

### EXEMPLES

### EXEMPLE 1

### Synthèses du composé 1,3,3-triméthylspiro [indoline-2,3'-[3H]-napht-[2,1-b]-[1,4]-oxazine], de structure :

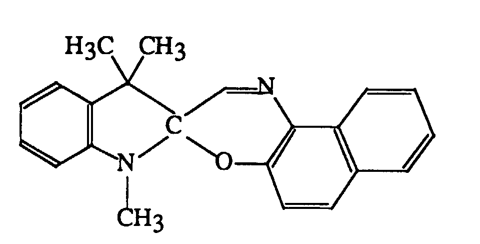

### Synthèse A

On chauffe le mélange composé de 0,173 g (1 mmole) de base de Fischer, 0,159 g (1mmole) de 1-amino-2-naphtol, de 5 ml de toluène et de 0,20 g (2,5 mmole) de diméthylsulfoxyde , pendant 6-7 heures, à 50-60°C. On chromatographie le produit de la réaction avec de l'oxyde d'aluminium. On cristallise le produit dans l'alcool.
Rendement : 0,24 g (72%)
Point de fusion : 125°C.

| Analyse élémentaire pour C₂₂H₂₀N₂O : | | | |
|---|---|---|---|
| | % C | % H | % N |
| Calculé | 80,46 | 6,12 | 8,81 |
| Trouvé | 80,53 | 6,25 | 8,77 |

Les spectres infrarouge et RMN¹H sont conformes à ceux du produit attendu.

### Synthèse B

On chauffe le mélange composé de 0,173 g (1 mmole) de base de Fischer, de 0,215 g (1,1 mmole) de chlorhydrate 1-amino-2-naphtol, de 0,150 g (1,5 mmole) de triéthylamine et de 0,195 g (2,5 mmole) de diméthylsulfoxyde, pendant 6 heures, à 50-60°C. On chromatographie le produit de la réaction avec de l'oxyde d'aluminiun. On cristallise le produit dans l'alcool.
Rendement : 0,19 g (60%)
Point de fusion: 125°C

Les spectres infrarouge et RMN¹H sont conformes à ceux du produit attendu.

### Synthèse C

Au mélange de 0,24 g (1,1 mmole) de chlorochromate de pyridinium et de 0,159 g (1 mmole) de 1-amino-2-naphtol, on ajoute une solution de 0,173 g (1 mmole) de base de Fischer dans 10 ml de chloroforme. on agite pendant 2 heures à température ambiante. On ajoute 0,5 g de triéthylamine. On agite la solution et on la filtre. On évapore le chloroforme. On dilue le résidu dans du toluène et on le chromatographie sur une colonne avec de l'oxyde d'aluminium. On cristallise le produit dans l'alcool.
Rendement : 0,20 g (61%)
Point de fusion : 125°C.

| Analyse élémentaire pour C₂₂H₂₀N₂O | | | |
|---|---|---|---|
| | % C | % H | % N |
| Calculé | 80,46 | 6,15 | 8,52 |
| Trouvé | 80,65 | 6,25 | 8,77 |

Les spectres infrarouge et RMN¹H sont conformes à ceux du produit attendu.

### Synthèse D

On ajoute 0,16 g (1,44 mmole) de bioxyde de sélénium à une solution de 0,173 g (1 mmole) de base de Fischer dans 6 ml de toluène. On agite le réactif pendant 1-1,5 heures, à température ambiante. On décante la solution de toluène du résidu. On y ajoute 0,159 g (1 mmole) de 1-amino-2-naphtol, et on le chauffe au bain-marie à une température de 50-100°C. On filtre la solution. On évapore en abaissant la pression jusqu'à un petit volume et on la chromatographie sur une colonne avec de l'oxyde d'aluminium. On cristallise le produit dans l'alcool.
Rendement: 0,17 g (52%)
Point de fusion : 125°C.

| Analyse élémentaire pour C₂₂H₂₀N₂O | | | |
|---|---|---|---|
| | % C | % H | % N |
| Calculé | 80,46 | 6,15 | 8,52 |
| Trouvé | 80,65 | 6,25 | 8,77 |

Les spectres infrarouge et RMN¹H sont conformes à ceux du produit attendu.

### EXEMPLE 2

### Synthèses du composé 1-propyle-3,3-diméthylspiro[indoline-2,3'-[3H]-napht-[2,1-b]-[1,4]-oxazine], de structure :

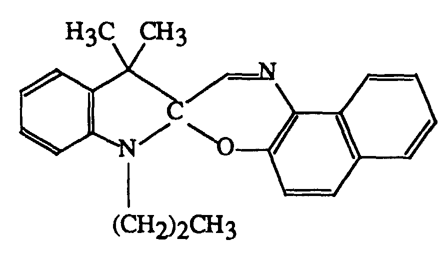

### Synthèse A

Le produit est obtenu selon la méthode A décrite dans l'exemple 1.
Rendement : 62%
Point de fusion : 123°C.

| Analyse élémentaire pour C₂₄H₂₄N₂O | | | |
|---|---|---|---|
| | % C | % H | % N |
| Calculé | 80,87 | 6,79 | 7,85 |
| Trouvé | 81,00 | 6,92 | 7,94 |

Les spectres infrarouge et RMN¹H sont conformes à ceux du produit attendu.

### Synthèse B

Le produit est obtenu selon la méthode C décrite dans l'exemple 1.
Rendement : 47%
Point de fusion : 123°C.

| Analyse élémentaire pour C₂₄H₂₄N₂O | | | |
|---|---|---|---|
| | % C | % H | % N |
| Calculé | 80,87 | 6,79 | 7,85 |
| Trouvé | 81,05 | 6,83 | 7,80 |

Les spectres infrarouge et RMN¹H sont conformes à ceux du produit attendu.

### Synthèse C

Le produit est obtenu selon la méthode D décrite dans l'exemple 1.
Rendement : 57%
Point de fusion : 123°C.

| Analyse élémentaire pour C₂₄H₂₄N₂O | | | |
|---|---|---|---|
| | % C | % H | % N |
| Calculé | 80,87 | 6,79 | 7,85 |
| Trouvé | 81,05 | 6,83 | 7,80 |

Les spectres infrarouge et RMN¹H sont conformes à ceux du produit attendu.

### EXEMPLE 3

### Synthèses du composé 1-hexyle-3,3-diméthylspiro[indoline-2,3'-[3H]-napht-[2,1-b]-[-1,4]-oxazine], de structure :

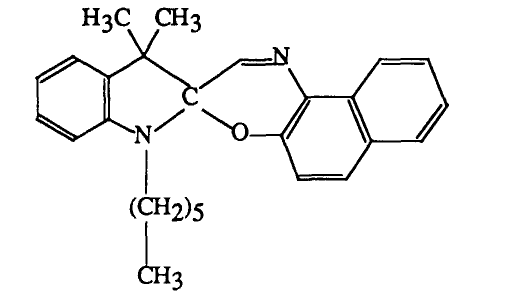

### Synthèse A

Le produit est obtenu selon la méthode B décrite dans l'exemple 1.
Rendement : 61%
Point de fusion : 90-91°C.

| Analyse élémentaire pour C₂₇H₃₀N₂O | | | |
|---|---|---|---|
| | %C | % H | % N |
| Calculé | 81,37 | 7,60 | 7,02 |
| Trouvé | 81,50 | 7,89 | 7,31 |

Les spectres infrarouge et RMN¹H sont conformes à ceux du produit attendu.

### Synthèse B

Le produit est obtenu selon la méthode C décrite dans l'exemple 1.
Rendement : 55%
Point de fusion : 90-91°C.

| Analyse élémentaire pour C₂₇H₃₀N₂O | | | |
|---|---|---|---|
| | % C | % H | % N |
| Calculé | 81,37 | 7,60 | 7,02 |
| Trouvé | 81,53 | 7,85 | 7,28 |

Les spectres infrarouge et RMN¹H sont conformes à ceux du produit attendu.

### Synthèse C

Le produit est obtenu selon la méthode D décrite dans l'exemple 1.
Rendement : 60%
Point de fusion : 90-91°C.

| Analyse élémentaire pour C₂₇H₃₀N₂O | | | |
|---|---|---|---|
| | % C | % H | % N |
| Calculé | 81,37 | 7,60 | 7,02 |
| Trouvé | 81,53 | 7,85 | 7,28 |

Les spectres infrarouge et RMN¹H sont conformes à ceux du produit attendu.

### EXEMPLE 4

### Synthèses du composé 1-benzyle-3,3-diméthylspiro[indolino-2,3'-[3H]-naphto-[2,1-b]-[1,4]-oxazine], de structure :

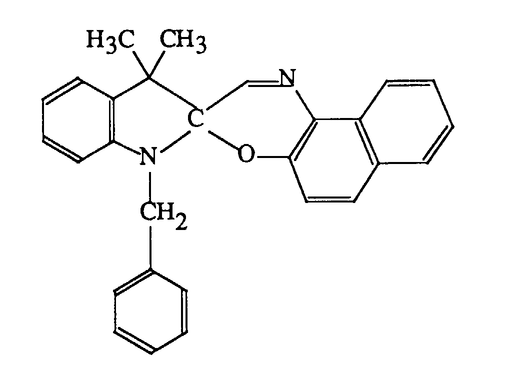

### Synthèse A

Le produit est obtenu selon la méthode A décrite dans l'exemple 1.
Rendement : 80%
Point de fusion : 192-193°C.

| Analyse élémentaire pour C₂₈H₂₄N₂O | | | |
|---|---|---|---|
| | % C | % H | % N |
| Calculé | 83,14 | 5,99 | 6,92 |
| Trouvé | 83,61 | 5,82 | 7,21 |

Les spectres infrarouge et RMN¹H sont conformes à ceux du produit attendu.

### Synthèse B

Le produit est obtenu selon la méthode C décrite dans l'exemple 1.
Rendement : 48%
Point de fusion : 192-193°C.

| Analyse élémentaire pour C₂₈H₂₄N₂O | | | |
|---|---|---|---|
| | % C | % H | % N |
| Calculé | 83,14 | 5,99 | 6,92 |
| Trouvé | 83,58 | 5,72 | 7,08 |

Les spectres infrarouge et RMN¹H sont conformes à ceux du produit attendu.

### Synthèse C

Le produit est obtenu selon la méthode D décrite dans l'exemple 1.
Rendement : 57%
Point de fusion : 192-193°C.

| Analyse élémentaire pour C₂₈H₂₄N₂O | | | |
|---|---|---|---|
| | % C | % H | % N |
| Calculé | 83,14 | 5,99 | 6,92 |
| Trouvé | 83,58 | 5,72 | 7,08 |

Les spectres infrarouge et RMN¹H sont conformes à ceux du produit attendu.

### EXEMPLE 5

### Synthèses du composé 1-[(3,4)-dichlorobenzyle]-3,3-diméthylspiro-[indoline-2,3-[3H]-napht-[2,1-b]-[1,4]-oxazine], de structure :

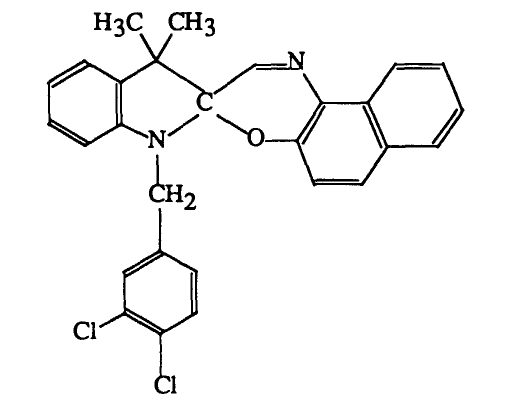

### Synthèse A

Le produit est obtenu selon la méthode A décrite dans l'exemple 1.
Rendement : 74%
Point de fusion : 177-178°C.

| Analyse élémentaire pour C₂₈H₂₂Cl₂N₂O | | | |
|---|---|---|---|
| | % C | % H | % N |
| Calculé | 71,04 | 4,69 | 5,91 |
| Trouvé | 71,43 | 4,85 | 6,08 |

Les spectres infrarouge et RMN¹H sont conformes à ceux du produit attendu.

### Synthèse B

Le produit est obtenu selon la méthode C décrite dans l'exemple 1
Rendement : 55%
Point de fusion : 177-178°C.

| Analyse élémentaire pour C₂₈H₂₂Cl₂N₂O | | | |
|---|---|---|---|
| | % C | % H | % N |
| Calculé | 71,04 | 4,69 | 5,91 |
| Trouvé | 71,52 | 4,92 | 6,01 |

Les spectres infrarouge et RMN¹H sont conformes à ceux du produit attendu.

### Synthèse C

Le produit est obtenu selon la méthode D décrite dans l'exemple 1.
Rendement : 60%
Point de fusion : 177-178°C.

| Analyse élémentaire pour C₂₈H₂₂Cl₂N₂O | | | |
|---|---|---|---|
| | % C | % H | % N |
| Calculé | 71,04 | 4,69 | 5,91 |
| Trouvé | 71,54 | 4,92 | 6,01 |

Les spectres infrarouge et PMR sont conformes au produit attendu.

### EXEMPLE 6

### Synthèse du 1,3,3-triméthylspiro[indoline-2,3'-[3H]-phénanthro-[9,10-B]-[1,4]-oxazine], de structure :

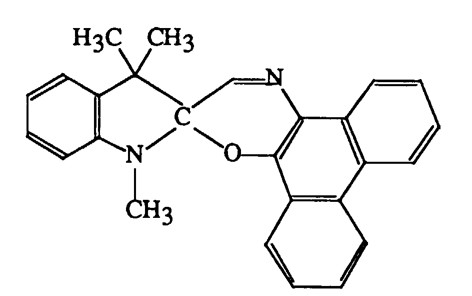

On chauffe à 50-60°C le mélange composé de 0,148 g (0,85 mmole) de base de Fischer, de 0,23 g (0,93 mmole) de chlorhydrate 9-amino-10-phénanthrol, de 5 ml de toluène, de 0,17 g (2,1 mmole) de diméthylsulfoxyde et de 0,13 g (1,27 mmole) de triéthylamine, pendant 6 heures. On chromatographie avec de l'oxyde d'aluminium en prélevant la fraction de couleur bleue. Le produit se cristallise dans l'alcool.
Rendement : 0,22 g (63%)
Point de fusion : 193-195°C.

| Analyse élémentaire pour C₂₆H₂₂N₂O | | | |
|---|---|---|---|
| | % C | % H | % N |
| Calculé | 82,52 | 5,87 | 7,39 |
| Trouvé | 82,68 | 5,82 | 7,48 |

Les spectres infrarouge et RMN¹H sont conformes à ceux du produit attendu.

### EXEMPLE 7

### Synthèses du composé 1,3,3-triméthylspiro[indoline-2,3'-[3H]-anthracéno-[2,1-b]-[1,4]-oxazine], de structure :

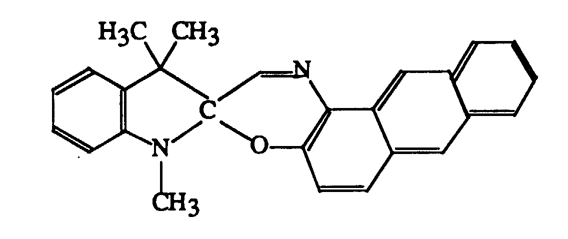

### Synthèse A

Le produit est obtenu selon la méthode B de l'exemple 1.
Rendement : 52%
Point de fusion : 186°C.

| Analyse élémentaire pour C₂₆H₂₂N₂O | | | |
|---|---|---|---|
| | % C | % H | % N |
| Calculé | 82,52 | 5,87 | 7,39 |
| Trouvé | 82,68 | 5,77 | 7,52 |

Les spectres infrarouge et RMN¹H sont conformes à ceux du produit attendu.

### Synthèse B

Le produit est obtenu selon la méthode C de l'exemple 1.
Rendement : 52%
Point de fusion : 186°C.

| Analyse élémentaire pour C₂₆H₂₂N₂O | | | |
|---|---|---|---|
| | % C | % H | % N |
| Calculé | 82,52 | 5,87 | 7,39 |
| Trouvé | 82,61 | 5,75 | 7,42 |

Les spectres infrarouge et RMN¹H sont conformes à ceux du produit attendu.

### Synthèse C

Le produit est obtenu selon la méthode D de l'exemple 1.
Rendement : 52%
Point de fusion : 186°C.

| Analyse élémentaire pour C₂₆H₂₂N₂O | | | |
|---|---|---|---|
| | % C | % H | % N |
| Calculé | 82,52 | 5,87 | 7,39 |
| Trouvé | 82,61 | 5,75 | 7,42 |

Les spectres infrarouge et RMN¹H sont conformes à ceux du produit attendu.

### COMPARAISON ENTRE DEUX METHODES DE PREPARATION DU

### 1,3,3-triméthylspiro[indoline-2,3'-[3H]-napht[2,1-b]-[1,4]-oxazine]

### I - Méthode classique :

A une solution de 1-nitroso-2-naphtol (0,190 g) (1,1 mmole) dans 10 ml de trichloréthylène, on ajoute goutte-à-goutte une solution de 2-méthylène-1,3,3-triméthylindoline (0,173 g) (1,1 mmole) dans 5 ml de trichloréthylène. Le mélange est porté au reflux pendant 3 heures, puis on évapore et purifie sur colonne de silice (100% dichlorométhane). La spironaphtoxazine attendue est recristallisée dans l'éthanol.
Rendement : 53%
Point de fusion : 125°C.

### Il - Méthode selon l'invention :

A un mélange de 2-méthylène-1,3,3-triméthylindoline (0,173 g) (1,1 mmole), sulfate de calcium anhydre (0,2 g), diméthylsulfoxyde (0,117 g) (1,5 mmole), hydrogénocarbonate de sodium (0,25 g), 1-amino-2-naphtol (0,175 g) (1,1 mmole), on ajoute 5 ml de toluène et laisse 12 heures sous agitation à 80°C. La réaction est suivie par TLC jusqu'à disparition d'une tache bleue de R_{f} = 0,3 sur plaque d'alumine neutre (hexane/chloroforme, 4:1). On filtre, lave avec du toluène chaud (2 ml), et évapore le filtrat. Le produit est ensuite purifié sur colonne de silice (90 hexane/10 acétate d'éthyle).
Rendement : 75%
Point de fusion : 125°C.

## Revendications

1. Procédé de préparation de composés photochromiques répondant à la structure spiro[indoline-[2,3']-benzoxazine] annélé, caractérisé par le fait qu'il comporte la condensation, en présence d'un agent oxydant, d'une base de Fischer du type 2-alkylidèneindoline sur un orthoaminophénol annélé.

2. Procédé selon la revendication 1, caractérisé par le fait que l'on mélange simultanément la base de Fischer, l'orthoaminophénol et l'agent oxydant.

3. Procédé selon la revendication 1 ou 2, caractérisé par le fait que l'on fait réagir dans une première étape l'agent oxydant sur la base de Fischer, puis que l'on additionne l'orthoaminophénol annélé au mélange réactionnel ainsi obtenu.

4. Procédé selon l'une quelconque des revendications 1 à 3, caractérisé par le fait que la base de Fischer du type alkylidèneindoline formée in situ dans le milieu réactionnel à partir d'un sel d'halogénure indoléninium correspondant, en présence d'une base.

5. Procédé selon l'une quelconque des revendications 1 à 4, caractérisé par le fait que l'orthoaminophénol annélé est formé in situ dans le milieu réactionnel à partir d'un sel d'addition d'acide correspondant en présence d'une base.

6. Procédé selon l'une quelconque des revendications 1 à 5, caractérisé par le fait qu'il est mis en oeuvre dans un milieu solvant.

7. Procédé selon l'une quelconque des revendications 1 à 6, caractérisé par le fait qu'il est mis en oeuvre dans un solvant choisi parmi le toluène et le chloroforme.

8. Procédé selon l'une quelconque des revendications 1 à 7, caractérisé par le fait que l'agent oxydant est un oxyde de métalloïde ou un oxyde de métal de transition.

9. Procédé selon la revendication 8, caractérisé par le fait que l'agent oxydant est choisi parmi le diméthylsulfoxyde, le bioxyde de selenium, le chlorochromate de pyridinium.

10. Procédé selon l'une quelconque des revendications 1 à 9, caractérisé par le fait qu'on mélange simultanément la base de Fischer, l'orthoaminophénol annélé et le diméthylsulfoxyde dans le toluène et que l'on chauffe ledit mélange à une température de 50-80°C.

11. Procédé selon la revendication 10, caractérisé par le fait qu'on ajoute dans le milieu réactionnel un agent déshydratant et/ou un agent activateur du diméthylsulfoxyde.

12. Procédé selon l'une quelconque des revendications 1 à 9, caractérisé par le fait que l'on fait réagir à température ambiante la base de Fischer avec le bioxyde de selenium dans le toluène et que l'on additionne ensuite l'orthoaminophénol annélé au milieu réactionnel, que l'on chauffe ensuite à une température de 50-100°C.

13. Procédé selon l'une quelconque des revendications 1 à 9, caractérisé par le fait qu'on mélange simultanément la base de Fischer, l'orthoaminophénol et le chlorochromate de pyridinium dans le chloroforme, à température ambiante puis, à la fin de la réaction de condensation de la base de Fischer avec l'orthoaminophénol annélé, qu'on additionne un réactif basique au mélange final.

14. Procédé selon l'une quelconque des revendications 1 à 13, caractérisé par le fait que l'on synthétise les composés répondant à la formule (III) : 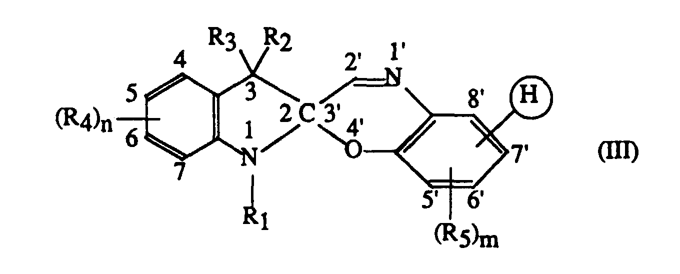 dans laquelle :
m est 1 ou 2;
n varie de 0 à 4;
R₁ représente :
i) un groupe alkyle de 1 à 16 atomes de carbone;
ii) un groupe allyle, phényle, arylalkyle mono- ou disubstitué par des substituants du type alkyle ou alcoxy de 1 à 6 atomes de carbone ou des atomes d'halogène tels que le chlore;
iii) un groupe alicyclique éventuellement substitué;
iv) un groupe hydrocarbure aliphatique comportant dans sa chaîne un ou plusieurs hétéroatomes choisis parmi O, N ou S, et éventuellement une fonction acide, ester ou alcool;
- R₂ et R₃ peuvent, chacun et indépendamment l'un de l'autre, représenter un groupe C₁-C₈ alkyle, phényle, phényle mono- ou disubstitué par des groupes C₁-C₄ alkyle et/ou C₁-C₅ alcoxy ou peuvent être combinés pour former une chaîne cyclique de 6 à 8 atomes de carbone;
- R₄ et R₅ peuvent, chacun et indépendamment l'un de l'autre, représenter :
i) un atome d'hydrogène, une fonction amine NR'R", où R' et R" représentent chacun et indépendamment un atome d'hydrogène, un groupe alkyle, cycloalkyle, phényle ou un dérivé substitué de celui-ci; R' et R" peuvent se combiner pour former un cycloalkyle pouvant être substitué et contenir un ou plusieurs hétéroatomes;
ii) un groupe R, OR, SR, COR ou COOR, dans lequel R représente un atome d'hydrogène, un groupe alkyle de 1 à 6 atomes de carbone ou un groupe aryle ou hétéroaryle;
iii) un atome d'halogène, un groupe C₁-C₄ monohaloalkyle, l'halogène étant notamment Cl ou Br, ou un groupe C₁-C₄ polyhaloalkyle tel que CF₃;
iv) -NO₂, CN, SCN,
chacun des substituants R₄ pouvant être présent sur l'un quelconque des atomes de carbone convenables de la partie indoline du composé photochromique, en positions 4, 5, 6 et 7, quand l'autre est un atome d'hydrogène, et quand n=2, les substituants étant présents en positions 4 et 5, 5 et 6, 4 et 6 ou 6 et 7;
si m=1, R₅ peut désigner un cycle benzénique condensé en positions 5', 6';
H désigne un cycle benzénique ou naphtalénique condensé en 7', 8' ou un hétérocycle à 5 ou 6 chaînons comportant 1 à 3 hétéroatomes choisis parmi oxygène, soufre ou azote, pouvant être substitué par un ou plusieurs groupements alkyle, alcoxy, amino, aryle, aralkyle ou condensé par un noyau aromatique:
en faisant réagir une base de Fischer de formule (I) : 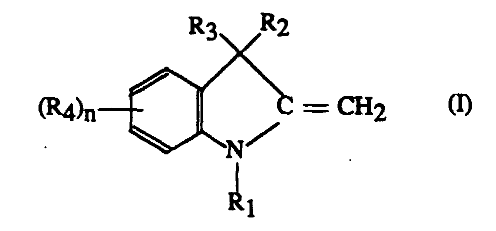 dans laquelle R₁, R₂, R₃, R₄ et n ont les mêmes significations indiquées ci-dessus, sur un orthoaminophénol annélé de formule suivante (II): 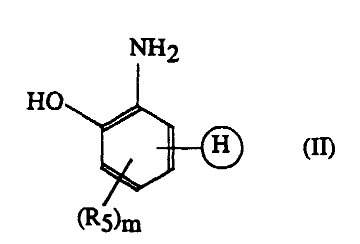 où R₅, H et m ont les mêmes significations indiquées ci-dessus, en présence de l'agent oxydant.

15. Procédé selon la revendication 14, caractérisé par le fait que l'on forme in situ la base de Fischer de formule (I) à partir d'un précurseur de formule (I') : 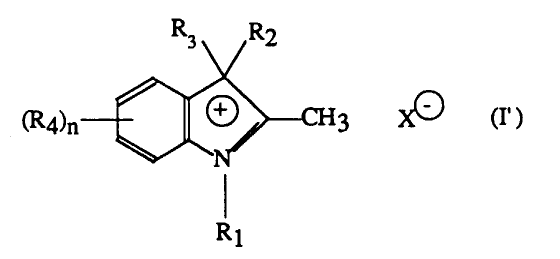 où R₁, R₂, R₃, R₄ et n ont les mêmes significations indiquées dans la revendication 10, et X désigne un atome d'halogène, en présence d'une base.

16. Procédé selon la revendication 14, caractérisé par le fait que l'on forme in situ l'orthoaminophénol de formule (II) à partir d'un précurseur de formule (II') : 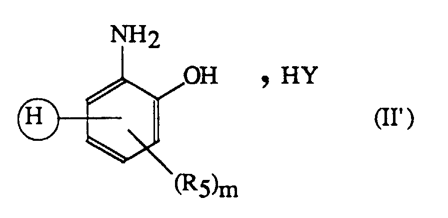 où R₅, H et m ont les mêmes significations indiquées ci-dessus, et Y désigne Cl⁻, HSO₄⁻, Br⁻, en présence d'une base.

17. Procédé selon l'une quelconque des revendications 1 à 16, caractérisé par le fait que l'on synthétise des composés de formule (III') : 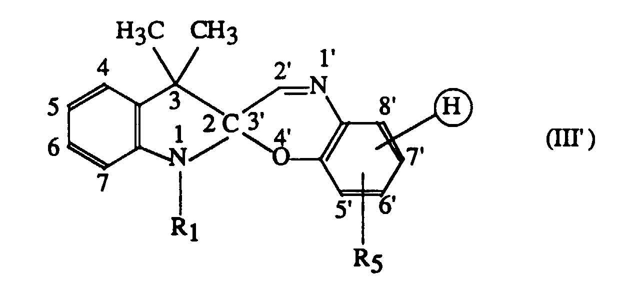 dans laquelle :
R₁ désigne un radical alkyle en C₁-C₈, benzyle, 3,4-dichlorobenzyle;
R₅ désigne un atome d'hydrogène ou un cycle benzénique condensé en positions 5', 6';
H désigne un cycle benzénique ou naphtalénique condensé en 7', 8' ou un hétérocycle à 5 ou 6 chaînons comportant 1 à 3 hétéroatomes choisis parmi oxygène, soufre et azote éventuellement substitué par un ou plusieurs groupes alkyle, alcoxy, amino, aryle, aralkyle ou condensé par un noyau aromatique,
en faisant réagir une base de Fischer de formule (IA) : 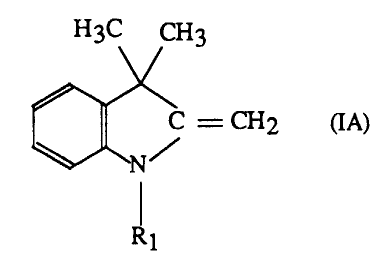 sur un orthoaminophénol annélé de formule (IIA) : 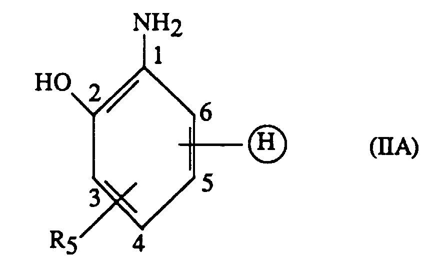 en présence de l'agent oxydant, les radicaux R₁, R₅ et H ayant les significations indiquées ci-dessus, et R₅ est condensé en 3,4 lorsqu'il désigne un noyau benzénique et H étant condensé en 5,6 lorsqu'il désigne un cycle benzénique ou naphtalénique.

18. Procédé selon la revendication 17, caractérisé par le fait que l'on forme in situ la base de Fischer de formule (IA) à partir d'un précurseur de formule (IB) : 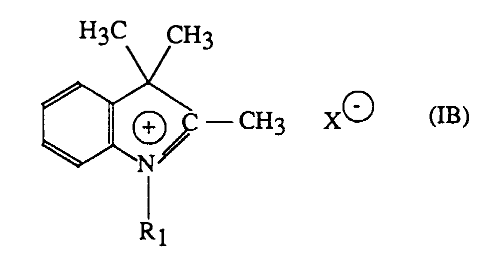 ou que l'on forme in situ l'orthoaminophénol de formule (IIA) à partir d'un précurseur de formule (IIB) : 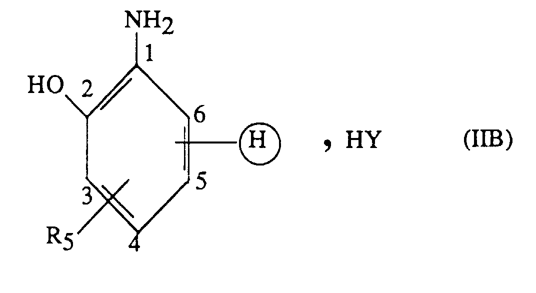 dans lesquelles :
R₁, R₅ ont les mêmes significations indiquées dans la revendication 14;
X désigne un halogène; et
Y désigne Cl⁻, Br⁻, HSO₄⁻.

## Patentansprüche

1. Verfahren zur Herstellung von fotochromen Verbindungen mit anellierter Spiro[indolin-[2,3']-benzoxazin]-Struktur, dadurch gekennzeichnet, daß es die Kondensation einer Fischerschen Base vom 2-Alkylidenindolintyp an ein anelliertes Orthoaminophenol in Gegenwart eines Oxidationsmittels umfaßt.

2. Verfahren nach Anspruch 1, dadurch gekennzeichnet, daß die Fischersche Base, das Orthoaminophenol und das Oxidationsmittel gleichzeitig gemischt werden.

3. Verfahren nach Anspruch 1 oder 2, dadurch gekennzeichnet, daß in einer ersten Stufe das Oxidationsmittel mit der Fischerschen Base reagieren gelassen wird und dann das anellierte Orthophenol der so erhaltenen Reaktionsmischung zugegeben wird.

4. Verfahren nach einem der Ansprüche 1 bis 3, dadurch gekennzeichnet, daß die Fischersche Base vom Alkylidenindolintyp aus einem entsprechenden Indoleniniumhalogenid in Anwesenheit einer Base in situ im Reaktionsmedium gebildet wird.

5. Verfahren nach einem der Ansprüche 1 bis 4, dadurch gekennzeichnet, daß das anellierte Orthoaminophenol aus einem entsprechenden Säureadditionssalz in Gegenwart einer Base in situ im Reaktionsmedium gebildet wird.

6. Verfahren nach einem der Ansprüche 1 bis 5, dadurch gekennzeichnet, daß es in einem Lösungsmittel-Medium durchgeführt wird.

7. Verfahren nach einem der Ansprüche 1 bis 6, dadurch gekennzeichnet, daß es in einem Lösungsmittel ausgewählt aus Toluol und Chloroform durchgeführt wird.

8. Verfahren nach einem der Ansprüche 1 bis 7, dadurch gekennzeichnet, daß das Oxidationsmittel ein Nichtmetalloxid oder ein Übergangsmetalloxid ist.

9. Verfahren nach Anspruch 8, dadurch gekennzeichnet, daß das Oxidationsmittel ausgewählt ist aus Dimethylsulfoxid, Selendioxid und Pyridiniumchlorchromat.

10. Verfahren nach einem der Ansprüche 1 bis 9, dadurch gekennzeichnet, daß die Fischersche Base, das anellierte Orthoaminophenol und das Dimethylsulfoxid in Toluol gleichzeitig gemischt werden und die Mischung auf eine Temperatur von 50-80°C erhitzt wird.

11. Verfahren nach Anspruch 10, dadurch gekennzeichnet, daß ein Dehydrationsmittel und/oder ein Dimethylsulfoxidaktivator dem Reaktionsmedium zugegeben werden.

12. Verfahren nach einem der Ansprüche 1 bis 9, dadurch gekennzeichnet, daß die Fischersche Base in Toluol mit Selendioxid bei Umgebungstemperatur reagieren gelassen wird und danach das anellierte Orthoaminophenol dem Reaktionsmedium zugegeben wird, welches anschließend auf eine Temperatur von 50-100°C erhitzt wird.

13. Verfahren nach einem der Ansprüche 1 bis 9, dadurch gekennzeichnet, daß die Fischersche Base, das Orthoaminophenol und das Pyridiniumchlorchromat in Chloroform bei Umgebungstemperatur gleichzeitig gemischt werden und nach Ende der Kondensationsreaktion der Fischerschen Base mit dem anellierten Orthoaminophenol ein basisches Reagens zur Endmischung zugegeben wird.

14. Verfahren nach einem der Ansprüche 1 bis 13, dadurch gekennzeichnet, daß die Verbindungen der Formel (III) synthetisiert werden: 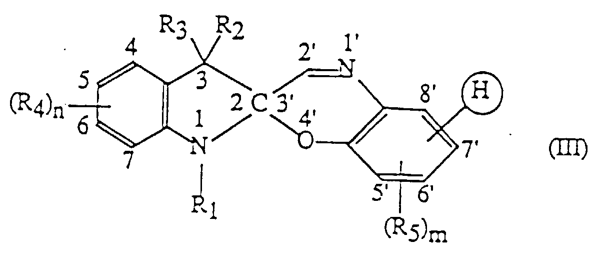 worin:
m 1 oder 2 ist;
n von 0 bis 4 variiert;
R₁ darstellt:
i) eine Alkylgruppe mit 1 bis 16 Kohlenstoffatomen;
ii) eine durch Substituenten vom Alkyl- oder Alkoxytyp mit 1 bis 6 Kohlenstoffatomen oder Halogenatome, wie Chlor, mono- oder disubstituierte Allyl-, Phenyl- oder Arylalkylgruppe;
iii) eine gegebenenfalls substituierte alizyklische Gruppe;
iv) eine aliphatische Kohlenwasserstoffgruppe mit einem oder mehreren Heteroatomen ausgewählt aus O, N oder S in ihrer Kette und gegebenenfalls einer Säure-, Ester- oder Alkoholfunktion;
R₂ und R₃ jeweils unabhängig voneinander eine C₁-C₈-Alkylgruppe, Phenylgruppe oder durch C₁-C₄-Alkyl- und/oder C₁-C₅-Alkoxygruppen mono- oder disubstituierte Phenylgruppe darstellen oder zur Bildung einer zyklischen Kette mit 6 bis 8 Kohlenstoffatomen verbunden sein können;
R₄ und R₅ jeweils unabhängig voneinander darstellen können:
i) ein Wasserstoffatom, eine Aminfunktion NR'R", worin R' und R" jeweils unabhängig voneinander für ein Wasserstoffatom, eine Alkyl-, Zykloalkyl-, Phenylgruppe oder ein substituiertes Derivat derselben stehen; R' und R" können verbunden sein, um ein Zykloalkyl zu bilden, welches substituiert sein und ein oder mehrere Heteroatome enthalten kann;
ii) eine Gruppe R, OR, SR, COR oder COOR, worin R ein Wasserstoffatom, eine Alkylgruppe mit 1 bis 6 Kohlenstoffatomen oder eine Aryl- oder Heteroarylgruppe ist;
iii) ein Halogenatom, eine C₁-C₄-Monohalogenalkylgruppe, wobei das Halogen insbesondere Cl oder Br ist, oder eine C₁-C₄-Polyhalogenalkylgruppe wie CF₃;
iv) -NO₂, CN, SCN,
wobei jeder der Substituenten R₄ an irgendeinem geeigneten Kohlenstoffatom des Indolinteils der fotochromen Verbindung in der Position 4, 5, 6 und 7 anwesend sein kann, wenn der andere ein Wasserstoffatom ist, und, wenn n = 2, die Substituenten an den Positionen 4 und 5, 5 und 6, 4 und 6 oder 6 und 7 vorhanden sind;
R₅, wenn m = 1, einen an der Position 5', 6' kondensierten Benzolring bezeichnen kann;
H einen an 7', 8' kondensierten Benzol- oder Naphthalinring oder einen 5- oder 6-gliedrigen Heterozyklus mit 1 bis 3 Heteroatomen ausgewählt aus Sauerstoff, Schwefel oder Stickstoff, der durch eine oder mehrere Alkyl-, Alkoxy-, Amino-, Aryl-, Aralkylgruppe(n) substituiert oder mit einem aromatischen Kern kondensiert sein kann, bezeichnet:
indem eine Fischersche Base der Formel (I): 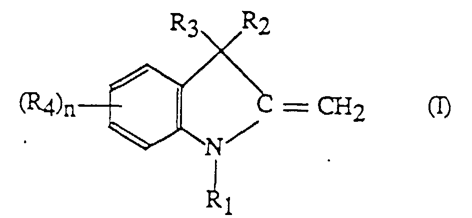 worin R₁, R₂, R₃, R₄ und n die oben angegebenen Bedeutungen haben, mit einem anellierten Orthoaminophenol der folgenden Formel (II): 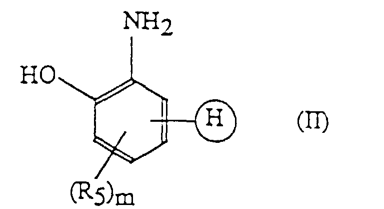 worin R₅, m und H die oben angegebenen Bedeutungen haben, in Gegenwart des Oxdiationsmittels reagieren gelassen wird.

15. Verfahren nach Anspruch 14, dadurch gekennzeichnet, daß die Fischersche Base der Formel (I) aus einer Vorstufe der Formel (I'): 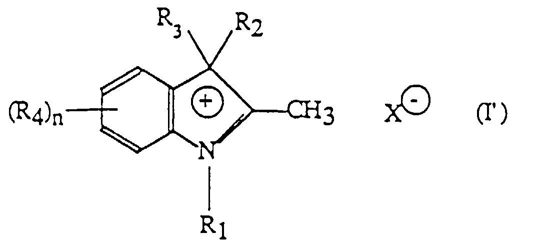 worin R₁, R₂, R₃, R₄ und n die in Anspruch 10 angegebenen Bedeutungen haben und X ein Halogenatom darstellt, in Gegenwart einer Base in situ gebildet wird.

16. Verfahren nach Anspruch 14, dadurch gekennzeichnet, daß das Orthoaminophenol der Formel (II) aus einer Vorstufe der Formel (II'): 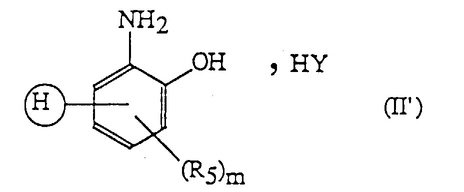 worin R₅, H und m die oben angegebenen Bedeutungen haben und Y für Cl⁻, HSO₄⁻ oder Br⁻ steht, in Gegenwart einer Base in situ gebildet wird.

17. Verfahren nach einem der Ansprüche 1 bis 16, dadurch gekennzeichnet, daß Verbindungen der Formel (III') synthetisiert werden: 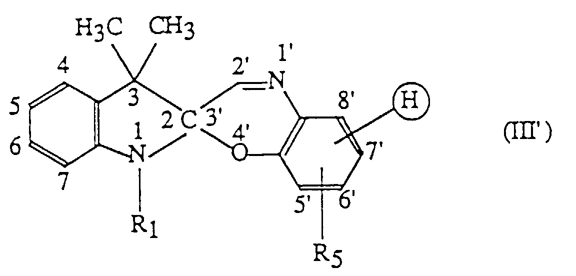 worin:
R₁ eine C₁-C₈-Alkyl-, Benzyl- oder 3,4-Dichlorbenzylgruppe bezeichnet;
R₅ ein Wasserstoffatom oder einen an den Positionen 5', 6' kondensierten Benzolring bezeichnet;
H einen an 7', 8' kondensierten Benzol- oder Naphthalinring oder einen 5- oder 6-gliedrigen Heterozyklus mit 1 bis 3 Heteroatomen ausgewählt aus Sauerstoff, Schwefel oder Stickstoff, welcher gegebenenfalls durch eine oder mehrere Alkyl-, Alkoxy-, Amino-, Aryl- oder Aralkylgruppe(n) substituiert oder mit einem aromatischen Kern kondensiert ist, bezeichnet,
indem eine Fischersche Base der Formel (IA): 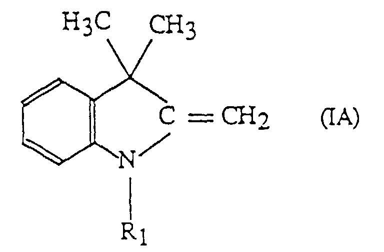 mit einem anellierten Orthoaminophenol der Formel (IIA): 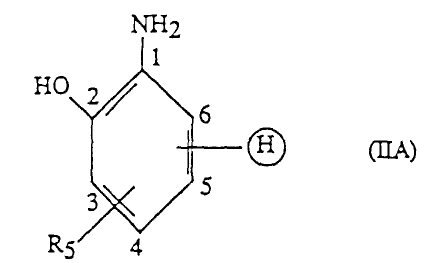 in Gegenwart des Oxidationsmittels reagieren gelassen wird, wobei die Reste R₁, R₅ und H die oben angegebenen Bedeutungen haben und R₅ an 3, 4 kondensiert ist, wenn es einen Benzolring bezeichnet, und H an 5, 6 kondensiert ist, wenn es einen Benzol- oder Naphthalinring bezeichnet.

18. Verfahren nach Anspruch 17, dadurch gekennzeichnet, daß die Fischersche Base der Formel (IA) aus einer Vorstufe der Formel (IB): 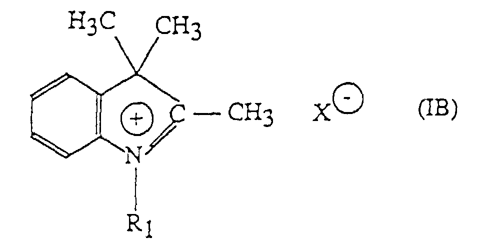 in situ gebildet wird und das Orthoaminophenol der Formel (IIA) aus einer Vorstufe der Formel (IIB): 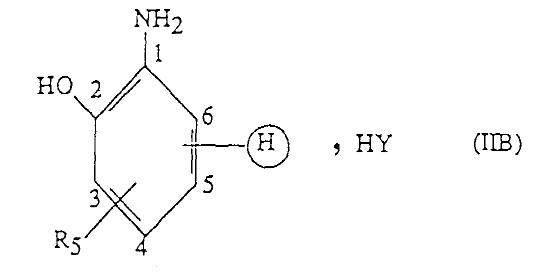 worin:
R₁, R₅ die in Anspruch 14 angegebenen Bedeutungen haben;
X ein Halogen bezeichnet; und
Y für Cl⁻, Br⁻ oder HSO₄⁻ steht,
in situ gebildet wird.

## Claims

1. Process for the preparation of photochromic compounds corresponding to the annelated spiro[indoline-[2,3']benzoxazine] structure, characterized in that it includes the condensation, in the presence of an oxidizing agent, of a Fischer base of the 2-alkylidene-indoline type onto an annelated ortho-aminophenol.

2. Process according to Claim 1, characterized in that the Fischer base, the ortho-aminophenol and the oxidizing agent are mixed together simultaneously.

3. Process according to Claim 1 or 2, characterized in that the oxidizing agent is reacted with the Fischer base in a first step and the annelated ortho-aminophenol is then added to the reaction mixture thus obtained.

4. Process according to any one of Claims 1 to 3, characterized in that the Fischer base of the alkylidene-indoline type [lacuna] formed in situ in the reaction medium from a corresponding indoleninium halide salt, in the presence of a base.

5. Process according to any one of Claims 1 to 4, characterized in that the annelated ortho-aminophenol is formed in situ in the reaction medium from an addition salt of the corresponding acid in the presence of a base.

6. Process according to any one of Claims 1 to 5, characterized in that it is carried out in a solvent medium.

7. Process according to any one of Claims 1 to 6, characterized in that it is carried out in a solvent chosen from toluene and chloroform.

8. Process according to any one of Claims 1 to 7, characterized in that the oxidizing agent is an oxide of a metalloid or an oxide of a transition metal.

9. Process according to Claim 8, characterized in that the oxidizing agent is chosen from dimethyl sulfoxide, selenium dioxide and pyridinium chlorochromate.

10. Process according to any one of Claims 1 to 9, characterized in that the Fischer base, the annelated ortho-aminophenol and the dimethyl sulfoxide are mixed together simultaneously in toluene and the said mixture is heated to a temperature of 50-80°C.

11. Process according to Claim 10, characterized in that a dehydrating agent and/or a dimethyl sulfoxide-activating agent is added to the reaction medium.

12. Process according to any one of Claims 1 to 9, characterized in that the Fischer base is reacted with the selenium dioxide in toluene at room temperature and the annelated ortho-aminophenol is then added to the reaction medium, which is then heated to a temperature of 50-100°C.

13. Process according to any one of Claims 1 to 9, characterized in that the Fischer base, the ortho-aminophenol and the pyridinium chlorochromate in chloroform are mixed together simultaneously at room temperature and at the end of the condensation reaction of the Fischer base with the annelated ortho-aminophenol, a basic reactant is then added to the final mixture.

14. Process according to any one of Claims 1 to 13, characterized in that compounds are synthesized corresponding to formula (III): 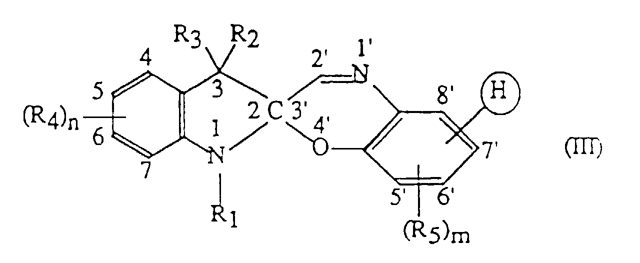 in which:
m is 1 or 2;
n ranges from 0 to 4;
R₁ represents:
i) an alkyl group of 1 to 16 carbon atoms;
ii) an allyl or phenyl group, an arylalkyl group mono- or disubstituted with substituents of the alkyl or alkoxy type of 1 to 6 carbon atoms, or halogen atoms such as chlorine;
iii) an optionally substituted alicyclic group;
iv) an aliphatic hydrocarbon group containing one or more hetero atoms chosen from O, N or S in its chain, and possibly an acid, ester or alcohol function;
- R₂ and R₃ can, each independently of each other, represent a C₁-C₈ alkyl group, a phenyl group, a phenyl group mono- or disubstituted with C₁-C₄ alkyl and/or C₁-C₅ alkoxy groups or may be combined to form a cyclic chain of 6 to 8 carbon atoms;
- R₄ and R₅ can, each independently of each other, represent:
i) a hydrogen atom, an amine function NR'R", where R' and R" each independently represent a hydrogen atom, an alkyl, cycloalkyl or phenyl group or a substituted derivative thereof; R' and R" may combine to form a cycloalkyl which may be substituted and contain one or more hetero atoms;
ii) a group R, OR, SR, COR or COOR, in which R represents a hydrogen atom, an alkyl group of 1 to 6 carbon atoms or an aryl or heteroaryl group;
iii) a halogen atom, a C₁-C₄ monohaloalkyl group, the halogen being Cl or Br in particular, or a C₁-C₄ polyhaloalkyl group such as CF₃;
iv) -NO₂, CN, SCN,
it being possible for each of the substituents R₄ to be present on any one of the suitable carbon atoms in the indoline part of the photochromic compound, in positions 4, 5, 6 and 7, when the other is a hydrogen atom, and when n=2, the substituents are present in positions 4 and 5, 5 and 6, 4 and 6 or 6 and 7;
if m=1, R₅ can also denote a 5',6'-fused benzene ring;
H denotes a 7',8'-fused benzene or naphthalene ring system or a 5- or 6-membered heterocycle containing 1 to 3 hetero atoms chosen from oxygen, sulfur and nitrogen, which may be substituted with one or more alkyl, alkoxy, amino, aryl or aralkyl groups or fused to an aromatic ring:
by reacting a Fischer base of formula (I): 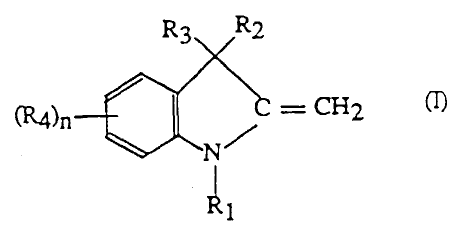 in which R₁, R₂, R₃, R₄ and n have the meanings indicated above, with an annelated ortho-aminophenol of formula (II) below: 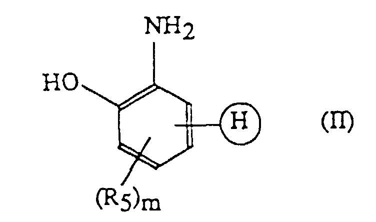 where R₅, H and m have the meanings indicated above, in the presence of the oxidizing agent.

15. Process according to Claim 14, characterized in that the Fischer base of formula (I) is formed in situ from a precursor of formula (I'): 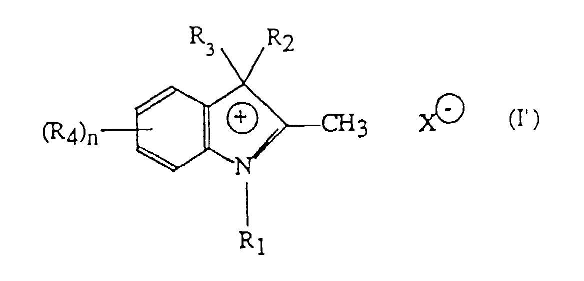 in which R₁, R₂, R₃, R₄ and n have the meanings indicated in Claim 10, and X denotes a halogen atom, in the presence of a base.

16. Process according to Claim 14, characterized in that the ortho-aminophenol of formula (II) is formed in situ from a precursor of formula (II'): 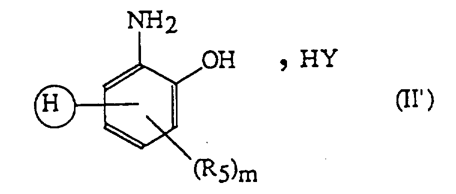 where R₅, H and m have the same meanings indicated above, and Y denotes Cl⁻, HSO₄⁻ or Br⁻, in the presence of a base.

17. Process according to any one of Claims 1 to 16, characterized in that compounds of formula (III'): 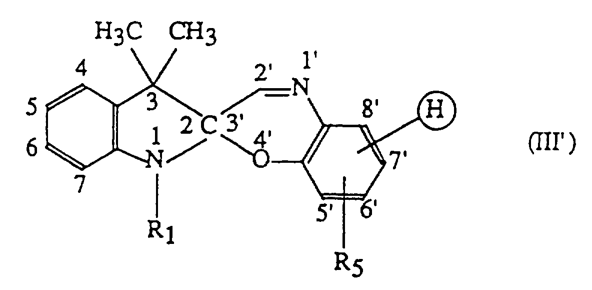 in which:
R₁ denotes a C₁-C₈ alkyl radical, a benzyl radical or a 3,4-dichlorobenzyl radical;
R₅ denotes a hydrogen atom or a 5',6'-fused benzene ring;
H denotes a 7',8'-fused benzene or naphthalene ring system or a 5- or 6-membered heterocycle containing 1 to 3 hetero atoms chosen from oxygen, sulfur and nitrogen, optionally substituted with one or more alkyl, alkoxy, amino, aryl or aralkyl groups or fused to an aromatic ring,
are synthesized by reacting a Fischer base of formula (IA) : 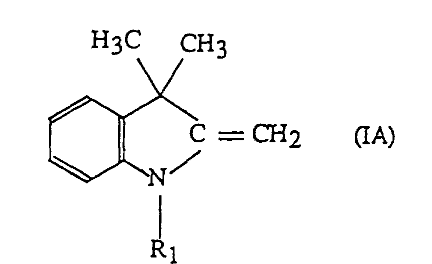 with an annelated ortho-aminophenol of formula (IIA) : 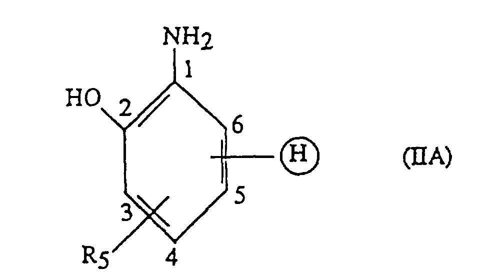 in the presence of the oxidizing agent, the radicals R₁, R₅ and H having the meanings indicated above, and R₅ is 3,4-fused when it denotes a benzene ring and H being 5,6-fused when it denotes a benzene or naphthalene ring system.

18. Process according to Claim 17, characterized in that the Fischer base of formula (IA) is formed in situ from a precursor of formula (IB): 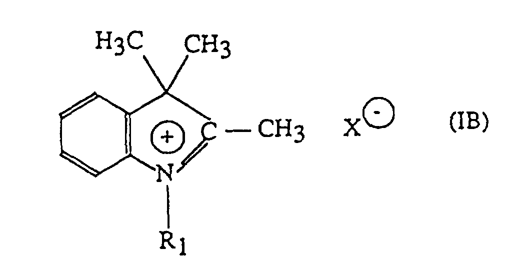 or in that the ortho-aminophenol of formula (IIA) is formed in situ from a precursor of formula (IIB) : 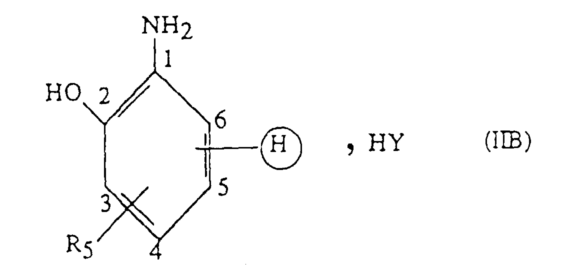 in which:
R₁ and R₅ have the same meanings indicated in Claim 14;
X denotes a halogen; and
Y denotes Cl⁻, Br⁻ or HSO₄⁻.
